(19) **Europäisches Patentamt / European Patent Office / Office européen des brevets**

(11) **EP 3 885 081 B1**

(12) **EUROPEAN PATENT SPECIFICATION**

(45) Date of publication and mention
of the grant of the patent:
**28.05.2025 Bulletin 2025/22**

(21) Application number: **19886893.7**

(22) Date of filing: **08.11.2019**

(51) International Patent Classification (IPC):
***B25J 11/00*** *(2006.01)*      ***A61F 2/60*** *(2006.01)*
***A61F 2/70*** *(2006.01)*

(52) Cooperative Patent Classification (CPC):
**B62D 57/032; B25J 9/0006**

(86) International application number:
**PCT/JP2019/043800**

(87) International publication number:
**WO 2020/105462 (28.05.2020 Gazette 2020/22)**

(54) **LOAD REDUCTION DEVICE, LOAD REDUCTION METHOD, AND STORAGE MEDIUM FOR STORING PROGRAM THEREIN**

LASTREDUZIERUNGSVORRICHTUNG UND LASTREDUZIERUNGSVERFAHREN SOWIE SPEICHERMEDIUM ZUR SPEICHERUNG EINES PROGRAMMS DARAUF

DISPOSITIF DE RÉDUCTION DE CHARGE, PROCÉDÉ DE RÉDUCTION DE CHARGE ET SUPPORT DE STOCKAGE STOCKANT UN PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.11.2018 JP 2018219220**

(43) Date of publication of application:
**29.09.2021 Bulletin 2021/39**

(73) Proprietor: NEC Corporation
**108-8001 Tokyo (JP)**

(72) Inventor: **OOKOBA, Tadashi**
**Tokyo 108-8001 (JP)**

(74) Representative: **Vossius & Partner**
**Patentanwälte Rechtsanwälte mbB**
**Siebertstraße 3**
**81675 München (DE)**

(56) References cited:
WO-A1-2010/011848      JP-A- 2011 206 289
JP-A- 2013 138 848      JP-A- 2017 099 798
KR-B1- 101 317 354

## Description

TECHNICAL FIELD

The present invention relates to a load reduction device, a load reduction method, and a storage medium for storing a program therein.

BACKGROUND ART

There is known a load reduction device that performs assistance of a load such as a walking motion of a user and mitigates the load of luggage carried by the user, when worn by the user. When wearable by a person, the load reduction device is sometimes called a powered suit.

Some powered suits assist walking movement by driving a link mechanism provided on the user's legs by outputting torque from an actuator to assist muscle strength. Patent Document 1 discloses a powered suit that smoothly supports a load with little discomfort and shock by outputting torque at the timing of transition from a swing state to a stance state. Patent Document 2 relates to a walking aid device that assists a user's walking motion. Patent Document 3 relates to a power-assisted robotic device that assists a wearer in performing forceful works. Patent Document 4 relates to an exoskeleton adapted to decrease energy consumption of a user. Patent Document 5 relates to a method of controlling a walking assist torque for assisting a walking of a pedestrian. Patent Documents 2 to 5 describe a load reduction device with torque estimation means and stance/swing determination means on the basis of loads on soles of the legs and an angle at each of the joints of the legs.

CITATION LIST

Patent Literature

[Patent Document 1] Japanese Unexamined Patent Application Publication No. 2015-144787
[Patent Document 2] Japanese Unexamined Patent Application Publication No. 2017-099798
[Patent Document 3] Japanese Unexamined Patent Application Publication No. 2013-138848
[Patent Document 4] WO 2010/011848 A1
[Patent Document 5] KR 10 1317354 B

SUMMARY OF THE INVENTION

Problems to be solved by the Invention

However, while in the technique described in Patent Document 1, the torque is adjusted by using a correction coefficient according to the ratio of the floor reaction forces of the right leg and the left leg, in a single-leg bending and stretching motion and a single-leg jumping motion and the like, smooth switching is not possible due to being a single-leg movement. That is, it is not possible to smoothly reduce the load for every motion of the user.

Therefore, an example object of the present invention is to provide a load reduction device, a load reduction method, and a storage medium for storing a program therein that can solve the above-mentioned problems.

Means for Solving the Problems

The invention is specified by the independent claims. Preferred embodiments are defined in the dependent claims.

Advantageous Effects of Invention

According to the present invention, it is possible to provide smooth load reduction for all motions of the user.

BRIEF DESCRIPTION OF THE DRAWINGS

FIG. 1 is a perspective view showing a configuration of a powered suit according to an embodiment of the present invention.

FIG. 2 is a diagram showing a hardware configuration of a control device according to an embodiment of the present invention.

FIG. 3 is a function block diagram of a control device according to an embodiment of the present invention.

FIG. 4 is an operation block diagram showing an operation of a control device according to an embodiment of the present invention.

FIG. 5 is a diagram showing an example of a stance torque control model according to an embodiment of the present invention.

FIG. 6 is a diagram showing the detection result of each sensor and the drive mode of each actuator in accordance with a user state according to an embodiment of the present invention.

FIG. 7 is a graph showing an outline of a smoothing process according to an example.

FIG. 8 is a graph showing the transition of the output torque of the actuator according to the embodiment of the present invention.

FIG. 9 is a flowchart showing the processing of a powered suit according to an embodiment of the present invention.

FIG. 10 is a diagram showing a minimum configuration of the control device according to the embodiment of the present invention.

EXAMPLE EMBODIMENT

Hereinbelow, a load reduction device, a load reduction method, and a storage medium for storing a program therein according to an embodiment of the present invention will be described with reference to the drawings.

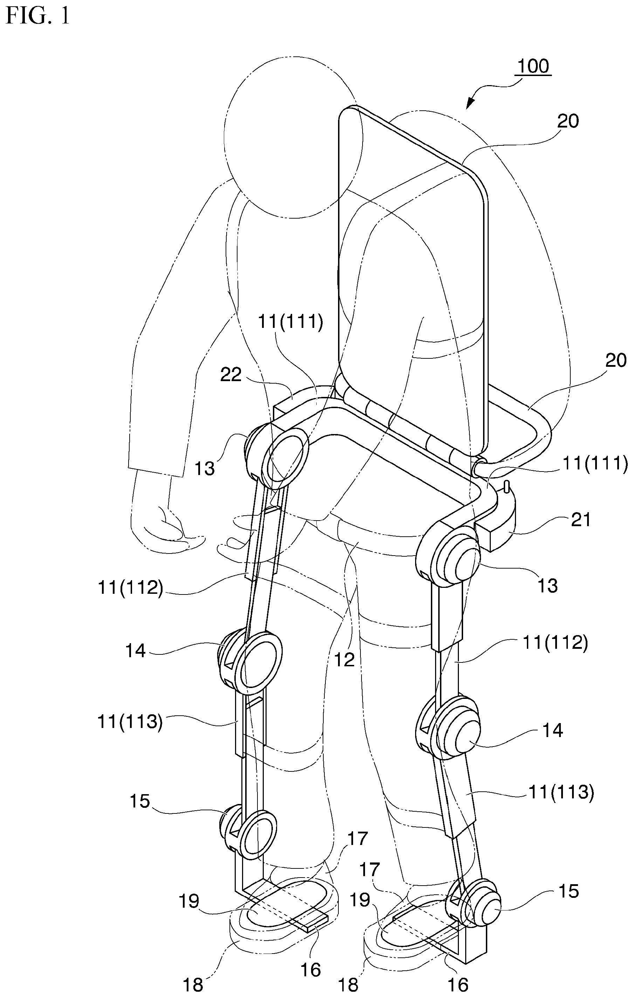

FIG. 1 is a diagram showing a configuration of a powered suit according to the present embodiment.

A powered suit 100 is one aspect of the load reduction device. The powered suit 100 is constituted by a skeleton portion 11, a belt 12, a hip actuator 13, a knee actuator 14, an ankle actuator 15, a shoe sole plate 16, a foot harness 17, a foot sole load sensor 18, a loading platform 20, a control device 21, a battery 22, a hip joint sensor 23, a knee joint sensor 24, an ankle joint sensor 25, and the like. The skeleton portion 11 is roughly classified into a first skeleton portion 111, a second skeleton portion 112, and a third skeleton portion 113 as an example.

As shown in FIG. 1, the powered suit 100 is configured as follows so as to support the loading platform 20, which is one aspect of the mechanism for holding luggage as an example. That is, the powered suit 100 is provided with the first skeleton portion 111, and the left and right hip actuators 13 are coupled rotatable to the first skeleton portion 111 and the second skeleton portion 112, which corresponds to the left or right thigh portion of the user wearing the powered suit 100, respectively. The left and right knee actuators 14 couple rotatable the corresponding second skeleton portion 112 on the left or right side and the corresponding third skeleton portion 113 along the left or right lower leg portion of the user wearing the powered suit 100. The ankle actuators 15 couple rotatable to the corresponding third skeleton portion 113 on the left or right side, and a corresponding shoe sole plate 16 provided on the back of the foot harness 17 on the left or right side of the user wearing the powered suit 100. The actuators 13, 14 and 15 are drive mechanisms that output torques that reduce the load on the user at each joint of each leg of the user.

The user who wears the powered suit 100 puts his/her left and right feet into the corresponding foot harnesses 17, and fixes the first skeleton portion 111 to the waist with the belt 12 so that the first skeleton portion 111 is closely attached to the waist. The powered suit 100 has a structure in which most of the load of the luggage and the load of the powered suit 100 is released to the ground surface in contact with the soles of the feet via the skeleton portion 11 and the actuators 13, 14, and 15. The user turns on the control device 21 of the powered suit 100. The control device 21 controls the actuators 13, 14, and 15 so as to transmit as much of the device weight as possible, which is the sum of the load of the luggage loaded on the loading platform 20 and the weight of the powered suit 100, to the walking surface via the skeleton portion 11 and the actuators 13, 14 and 15. Thereby, the powered suit 100 mitigates the burden such as the load of the luggage on the user who wears the powered suit 100 and performs various motions.

The hip joint sensor 23 is installed in the hip actuator 13, and detects the hip joint angle, that is, the angle formed between the first skeleton portion 111 and the second skeleton portion 112, by an encoder. The knee joint sensor 24 is installed in the knee actuator 14, and detects the knee joint angle, that is, the angle between the second skeleton portion 112 and the third skeleton portion 113, by the encoder. The ankle joint sensor 25 is installed in the ankle actuator 15, and detects the ankle joint angle, that is, the angle between the third skeleton portion 113 and the shoe sole plate 16, by the encoder. The joint sensors 23, 24, and 25 detect the angle of each joint of each leg of the user (hereinafter referred to as "joint angle").

The foot sole load sensors 18 detect the values of loads on the soles of the feet of each leg of the user. The foot sole load sensor 18 is provided so as to cover the entire sole of each foot so as to be able to measure the weight inside the foot harness 17 from the sole of the user. For example, the foot sole load sensor 18 is attached between the insole of the foot harness 17 and the shoe sole plate 16.

As an example, the foot sole load sensor 18 has electrodes arranged in a matrix on the front and back of a thin sheet-like insulator, measures the electrical resistance of the lattice points of the electrodes, and outputs the measured value to the control device 21. The control device 21 calculates the pressure applied to each lattice point and the load value on the entire surface of the sensor sheet on the basis of the electrical resistance value of each lattice point.

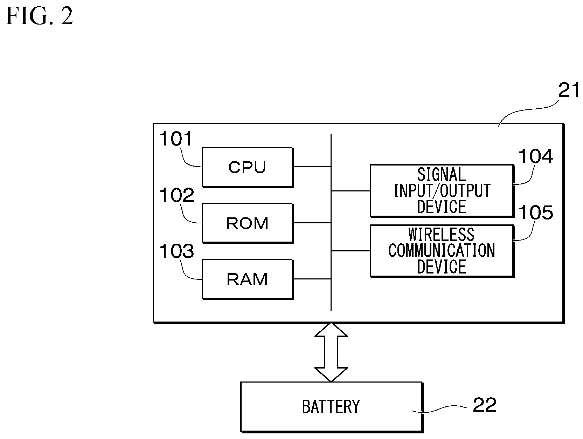

FIG. 2 is a diagram showing the hardware configuration of the control device.

As shown in this figure, the control device 21 is a computer provided with hardware such as a CPU (Central Processing Unit) 101, a ROM (Read Only Memory) 102, a RAM (Random Access Memory) 103, a signal input/output device 104, and a wireless communication device 105.

The signal input/output device 104 inputs signals output from the foot sole load sensor 18, the hip joint sensor 23, the knee joint sensor 24, and the ankle joint sensor 25. The signal input/output device 104 outputs control signals for controlling the hip actuator 13, the knee actuator 14, and the ankle actuator 15. The control device 21 operates by power supplied from the battery 22.

The wireless communication device 105 is communicatively connected with another device.

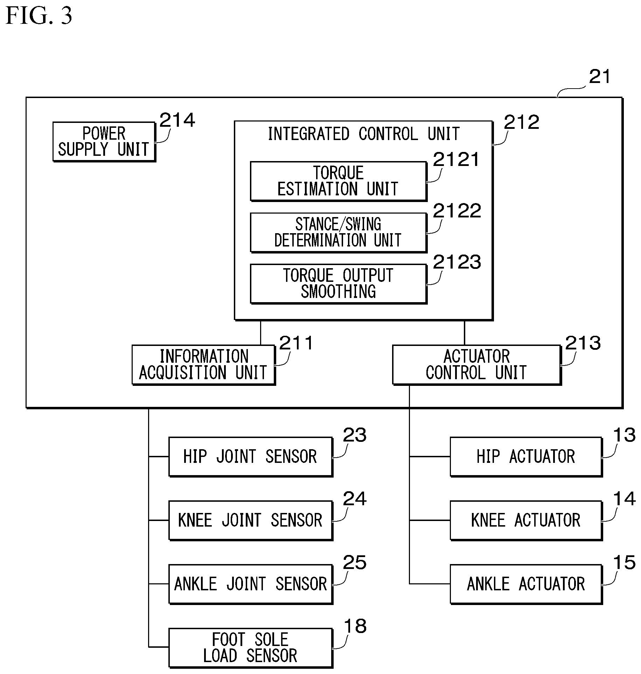

FIG. 3 is a function block diagram of the control device.

The control device 21 is activated based on the power supplied from the battery 22 by turning on the power button. The control device 21 executes the control program after startup. As a result, the control device 21 is provided with at least an information acquisition unit 211, an integrated control unit 212, an actuator control unit 213, and a power supply unit 214.

The information acquisition unit 211 acquires sensing information from the foot sole load sensor 18, the hip joint sensor 23, the knee joint sensor 24, and the ankle joint sensor 25. The sensing information of the foot sole load sensor 18 is sole load information indicating the detected load value. The sensing information of the hip joint sensor 23, the knee joint sensor 24, and the ankle joint sensor 25 is joint angle information indicating the detected joint angle.

The actuator control unit 213 controls the hip actuator 13, the knee actuator 14, and the ankle actuator 15.

When the power button is turned on, the power supply unit 214 supplies electric power from the battery 22 to each part of the control device 21.

The integrated control unit 212 is provided with a torque estimation unit 2121, a stance/swing determination unit 2122, and a torque output smoothing unit 2123.

The torque estimation unit 2121 calculates the stance torque output by the actuators 13, 14 and 15 during a stance period and the swing torque output by the actuators 13, 14 and 15 during a swing period on the basis of the values of loads on the soles of the feet of the legs and the angles of the respective joints of the legs. Specifically, the stance torque and the swing torque at each joint are calculated based on a control model in which the joints on the sagittal plane of the user are linked.

The stance/swing determination unit 2122 determines whether the states of a user's legs are stance or swing. Specifically, the stance/swing determination unit 2122, based on the load value applied to the sole of the foot of one leg, determines whether the state of the leg is the stance state or the swing state. For example, the stance/swing determination unit 2122 determines the state of the right leg on the basis of the load value applied to the sole of the foot of the right leg. The stance/swing determination unit 2122 determines the state of the left leg on the basis of the load value applied to the sole of the foot of the left leg.

When the state of each leg of the user has switched, the torque output smoothing unit 2123 transitions the torque output by the actuators 13, 14, and 15 based on the stance torque and the swing torque so as to become smooth according to the elapsed time. Specifically, the torque output smoothing unit 2123 provides a switching time for switching between a period in which the actuators 13, 14 and 15 output the stance torque and the period in which the actuators 13, 14 and 15 output the swing torque. The torque output smoothing unit 2123 transitions the magnitudes of the torques output by the actuators 13, 14 and 15 in the switching time according to the elapsed time. For example, the torque output smoothing unit 2123 adds the stance torque and the swing torque in accordance with a ratio according to the elapsed time to the switching time.

Subsequently, the operation of the control device 21 will be described in detail.

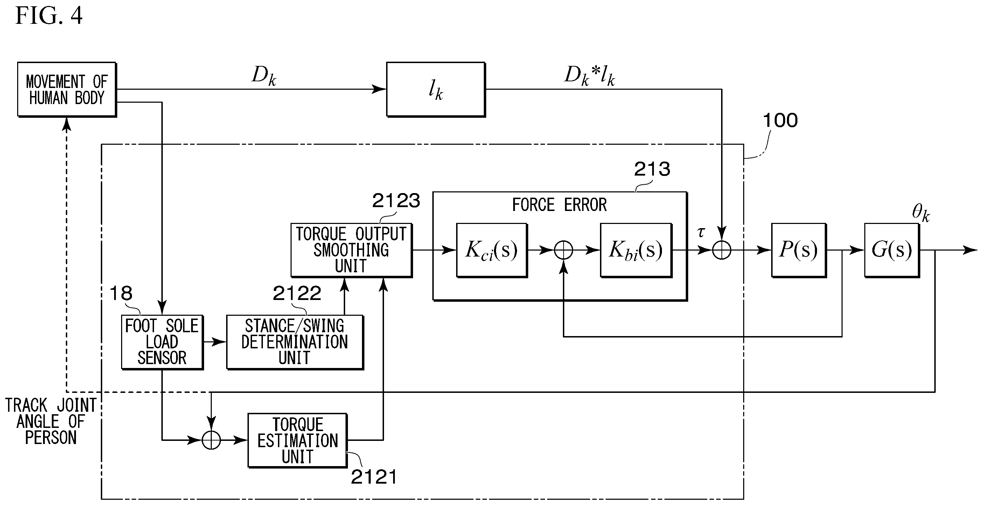

FIG. 4 is an operation block diagram showing the operation of the control device.

First, the torque estimation unit 2121 calculates the stance torque and the swing torque on the basis of values of loads detected by the foot sole load sensor 18 of each leg and the joint angles detected by the joint sensors 23, 24, and 25 of each leg.

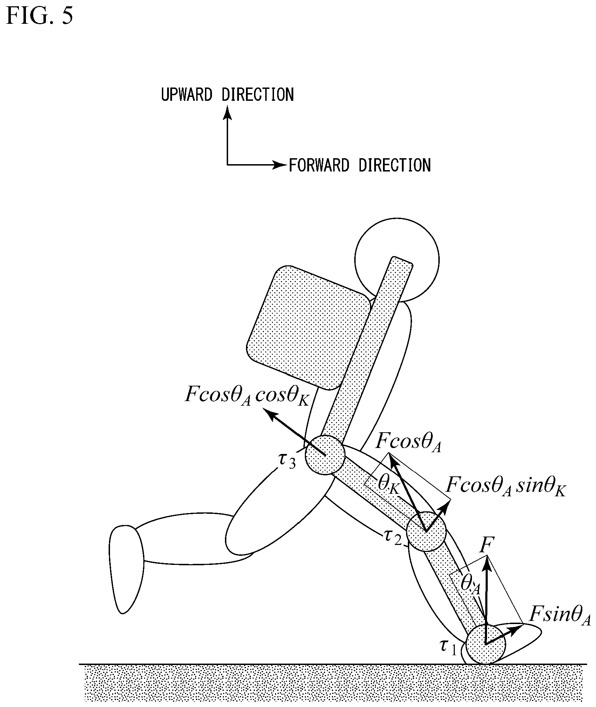

FIG. 5 is a diagram showing an example of a stance torque control model. As the control model in this embodiment, a three-link model in which the hip joint, knee joint, and ankle joint in the sagittal plane of the user are linked is adopted. For example, stance torque $\tau 3$ of the hip actuator 13 is a distributed load "$F\cos\theta_A\cos\theta_K$" in the rotational direction as seen from the upper body weight center axis. The value "F" is a floor reaction force value obtained by multiplying the weight of the user, the weight of the powered suit 100 and the luggage by the impact acceleration. The weight of the user, the weight of the powered suit 100, and the weight of the luggage are values measured in advance before the use of the powered suit 100. The impact acceleration may be estimated based on the load value detected by the foot sole load sensor 18, or may be specified based on the detection result of an acceleration sensor (not shown) provided on the foot harness 17.

The value "$\theta_A$" is the ankle joint angle detected by the ankle joint sensor 25. The value "$\theta_K$" is the knee joint angle detected by the knee joint sensor 24. FIG. 5 shows the distributed load "$F\cos\theta_A\sin\theta_K$" in the rotation direction seen from the hip joint axis, the distributed load "$F\cos\theta_A$" in the rotation direction seen from the knee joint axis, and the component force of the foot sole load sensor 18 (distributed load in the centripetal direction as seen from the joint axis) "$F\sin\theta_A$".

In the following, the direction in which the user travels is defined as the forward direction, and the direction opposite to the direction of gravity is defined as the upward direction. The XYZ coordinate system is defined with the forward direction as the X-axis direction, the upward direction orthogonal to the X-axis direction as the Z-axis direction, and the direction orthogonal to the X-axis direction and the Z-axis direction as the Y-axis direction.

Specifically, the torque estimation unit 2121 calculates the stance torque by the following equations of motion (1) to (8). The inertial matrix M is represented by the following Equation (1).

$$M = \begin{bmatrix} m_{11} & m_{12} & m_{13} & m_{14} & m_{15} \\ m_{21} & m_{22} & m_{23} & m_{24} & m_{25} \\ m_{31} & m_{32} & m_{33} & m_{34} & m_{35} \\ m_{41} & m_{42} & m_{43} & m_{44} & m_{45} \\ m_{51} & m_{52} & m_{53} & m_{54} & m_{55} \end{bmatrix} \quad \cdots(1)$$

Each element of the inertial matrix M is calculated by the following equations (1-1) to (1-25). Here, the value "$m_1$" is the mass around the ankle joint. The value "$m_2$" is the mass around the knee joint. The value "$m_3$" is the mass around the hip joint. The value "$l_1$" is the length from the foot to the knee. The value "$l_2$" is the length from the knee to the waist. The value "$l_3$" is the length of the upper body. The value "$l_{g1}$" is the length from the ankle joint to the center of gravity of the tibia. The value "$l_{g2}$" is the length from the knee joint to the center of gravity of the femur. The value "$l_{g3}$" is the length from the hip joint to the upper body center of gravity. The value "$I_1$" is the inertia around the ankle joint. The value "$I_2$" is the inertia around the knee joint. The value "$I_3$" is the inertia around the hip joint. The value "$\theta_1$" is the ankle joint angle. The value "$\theta_2$" is the knee joint angle. The value "$\theta_3$" is the hip joint angle.

The values of the mass $m_1$ around the ankle joint, the mass $m_2$ around the knee joint, the mass $m_3$ around the hip joint, the length $l_1$ from the foot to the knee, the length $l_2$ from the knee to the waist, the length $l_3$ of the upper body, the length $l_{g1}$ from the ankle joint to the center of gravity of the tibia, the length $l_{g2}$ from the knee joint to the center of gravity of the femur, the length $l_{g3}$ from the hip joint to the upper body center of gravity, the inertia $I_1$ around the ankle joint, the inertia $I_2$ around the knee joint and the inertia $I_3$ around the hip joint are set in advance in the control device 21.

$$m_{11} = m_1 + m_2 + m_3 \qquad \cdots(1\text{-}1)$$

$$m_{12} = 0 \qquad \cdots(1\text{-}2)$$

$$m_{13} = \left(m_1 l_{g1} + m_2 l_1 + m_3 l_1\right)\cos\theta_1 + \left(m_2 l_{g2} + m_3 l_2\right)\cos(\theta_1 + \theta_2) + m_3 l_{g3}\cos(\theta_1 + \theta_2 + \theta_3) \quad \cdots(1\text{-}3)$$

$$m_{14} = \left(m_2 l_{g2} + m_3 l_2\right)\cos(\theta_1 + \theta_2) + m_3 l_{g3}\cos(\theta_1 + \theta_2 + \theta_3) \qquad \cdots(1\text{-}4)$$

$$m_{15} = m_3 l_{g3}\cos(\theta_1 + \theta_2 + \theta_3) \qquad \cdots(1\text{-}5)$$

$$m_{21} = m_{21} \qquad \cdots(1\text{-}6)$$

$$m_{22} = m_1 + m_2 + m_3 \qquad \cdots(1\text{-}7)$$

$$m_{23} = \left(m_1 l_{g1} + m_2 l_1 + m_3 l_1\right)\sin\theta_1 + \left(m_2 l_{g2} + m_3 l_2\right)\sin(\theta_1 + \theta_2) + m_3 l_{g3}\sin(\theta_1 + \theta_2 + \theta_3) \quad \cdots(1\text{-}8)$$

$$m_{24} = \left(m_2 l_{g2} + m_3 l_2\right)\sin(\theta_1 + \theta_2) + m_3 l_{g3}\sin(\theta_1 + \theta_2 + \theta_3) \quad \cdots(1\text{-}9)$$

$$m_{25} = m_3 l_{g3}\sin(\theta_1 + \theta_2 + \theta_3) \quad \cdots(1\text{-}10)$$

$$m_{31} = m_{13} \quad \cdots(1\text{-}11)$$

$$m_{32} = m_{23} \quad \cdots(1\text{-}12)$$

$$m_{33} = m_1 l_{g1}{}^2 + (m_2 + m_3) l_1{}^2 + m_2 l_{g2}{}^2 + m_3 l_2{}^2 + m_3 l_{g3}{}^2 + \left(2 m_2 l_1 l_{g2} + 2 m_3 l_1 l_2\right)\cos\theta_2$$
$$+ 2 m_3 l_1 l_{g3}\cos(\theta_2 + \theta_3) + 2 m_3 l_2 l_{g3}\cos\theta_3 + I_1 + I_2 + I_3 \quad \cdots(1\text{-}13)$$

$$m_{34} = m_2 l_{g2}{}^2 + m_3 l_2{}^2 + m_3 l_{g3}{}^2 + \left(m_2 l_1 l_{g2} + m_3 l_1 l_2\right)\cos\theta_2 + m_3 l_1 l_{g3}\cos(\theta_2 + \theta_3)$$
$$+ 2 m_3 l_2 l_{g3}\cos\theta_3 + I_2 + I_3 \quad \cdots(1\text{-}14)$$

$$m_{35} = m_3 l_{g3}{}^2 + m_3 l_1 l_{g3}\cos(\theta_2 + \theta_3) + m_3 l_2 l_{g3}\cos\theta_3 + I_3 \quad \cdots(1\text{-}15)$$

$$m_{41} = m_{14} \quad \cdots(1\text{-}16)$$

$$m_{42} = m_{24} \quad \cdots(1\text{-}17)$$

$$m_{43} = m_{34} \quad \cdots(1\text{-}18)$$

$$m_{44} = m_2 l_{g2}{}^2 + m_3 l_2{}^2 + m_3 l_{g3}{}^2 + 2 m_3 l_2 l_{g3}\cos\theta_3 + I_2 + I_3 \quad \cdots(1\text{-}19)$$

$$m_{45} = m_3 l_{g3}{}^2 + m_3 l_2 l_{g3}\cos\theta_3 + I_3 \quad \cdots(1\text{-}20)$$

$$m_{51} = m_{15} \quad \cdots(1\text{-}21)$$

$$m_{52} = m_{25} \quad \cdots(1\text{-}22)$$

$$m_{53} = m_{35} \quad \cdots(1\text{-}23)$$

$$m_{54} = m_{45} \quad \cdots(1\text{-}24)$$

$$m_{55} = m_3 l_{g3}{}^2 + I_3 \quad \cdots(1\text{-}25)$$

The Coriolis force h is expressed by the following Equation (2).

$$h = \begin{bmatrix} h_1 & h_2 & h_3 & h_4 & h_5 \end{bmatrix}^T \quad \cdots(2)$$

Each element of the Coriolis force h is calculated by the following equations (2-1) to (2-5).

$$
\begin{aligned}
h_1 = &-\left\{\left(m_1 l_{g1} + m_2 l_2 + m_3 l_1\right)\sin\theta_1 + \left(m_2 l_{g2} + m_3 l_2\right)\sin(\theta_1 + \theta_2) + m_3 l_{g3}\sin(\theta_1 + \theta_2 + \theta_3)\right\}\dot{\theta}_1^2 \\
&- \left\{\left(m_2 l_{g2} + m_3 l_2\right)\sin(\theta_1 + \theta_2) + m_3 l_{g3}\sin(\theta_1 + \theta_2 + \theta_3)\right\}\dot{\theta}_2^2 \\
&- \left\{m_3 l_{g3}\sin(\theta_1 + \theta_2 + \theta_3)\right\}\dot{\theta}_3^2 \\
&- \left\{\left(2m_2 l_{g2} + 2m_3 l_2\right)\sin(\theta_1 + \theta_2) + 2m_3 l_{g3}\sin(\theta_1 + \theta_2 + \theta_3)\right\}\dot{\theta}_1\dot{\theta}_2 \\
&- \left\{2m_3 l_{g3}\sin(\theta_1 + \theta_2 + \theta_3)\right\}\dot{\theta}_1\dot{\theta}_3 \\
&- \left\{2m_3 l_{g3}\sin(\theta_1 + \theta_2 + \theta_3)\right\}\dot{\theta}_2\dot{\theta}_3 \quad\quad\cdots(2\text{-}1)
\end{aligned}
$$

$$
\begin{aligned}
h_2 = &\left\{\left(m_1 l_{g1} + m_2 l_2 + m_3 l_1\right)\cos\theta_1 + \left(m_2 l_{g2} + m_3 l_2\right)\cos(\theta_1 + \theta_2) + m_3 l_{g3}\cos(\theta_1 + \theta_2 + \theta_3)\right\}\dot{\theta}_1^2 \\
&+ \left\{\left(m_2 l_{g2} + m_3 l_2\right)\cos(\theta_1 + \theta_2) + m_3 l_{g3}\cos(\theta_1 + \theta_2 + \theta_3)\right\}\dot{\theta}_2^2 \\
&+ \left\{m_3 l_{g3}\cos(\theta_1 + \theta_2 + \theta_3)\right\}\dot{\theta}_3^2 \\
&+ \left\{\left(2m_2 l_{g2} + 2m_3 l_2\right)\cos(\theta_1 + \theta_2) + 2m_3 l_{g3}\cos(\theta_1 + \theta_2 + \theta_3)\right\}\dot{\theta}_1\dot{\theta}_2 \\
&+ \left\{2m_3 l_{g3}\cos(\theta_1 + \theta_2 + \theta_3)\right\}\dot{\theta}_1\dot{\theta}_3 \\
&+ \left\{2m_3 l_{g3}\cos(\theta_1 + \theta_2 + \theta_3)\right\}\dot{\theta}_2\dot{\theta}_3 \quad\quad\cdots(2\text{-}2)
\end{aligned}
$$

$$
\begin{aligned}
h_3 = &-\left\{\left(m_2 l_1 l_{g2} + m_3 l_1 l_2\right)\sin\theta_2 + m_3 l_1 l_{g3}\sin(\theta_2 + \theta_3)\right\}\dot{\theta}_2^2 \\
&- \left\{m_3 l_2 l_{g3}\sin\theta_3 + m_3 l_1 l_{g3}\sin(\theta_2 + \theta_3)\right\}\dot{\theta}_3^2 \\
&- \left\{\left(2m_2 l_1 l_{g2} + 2m_3 l_1 l_2\right)\sin\theta_2 + 2m_3 l_1 l_{g3}\sin(\theta_2 + \theta_3)\right\}\dot{\theta}_1\dot{\theta}_2 \\
&- \left\{2m_3 l_2 l_{g3}\sin\theta_3 + 2m_3 l_1 l_{g3}\sin(\theta_2 + \theta_3)\right\}\dot{\theta}_1\dot{\theta}_3 \\
&- \left\{2m_3 l_2 l_{g3}\sin\theta_3 + 2m_3 l_1 l_{g3}\sin(\theta_2 + \theta_3)\right\}\dot{\theta}_2\dot{\theta}_3 \quad\quad\cdots(2\text{-}3)
\end{aligned}
$$

$$
\begin{aligned}
h_4 = &\left\{\left(m_2 l_1 l_{g2} + m_3 l_1 l_2\right)\sin\theta_2 + m_3 l_1 l_{g3}\sin(\theta_2 + \theta_3)\right\}\dot{\theta}_1^2 \\
&- \left(m_3 l_2 l_{g3}\sin\theta_3\right)\dot{\theta}_3^2 \\
&- \left(2m_3 l_2 l_{g3}\sin\theta_3\right)\dot{\theta}_1\dot{\theta}_3 \\
&- \left(2m_3 l_2 l_{g3}\sin\theta_3\right)\dot{\theta}_2\dot{\theta}_3 \quad\quad\cdots(2\text{-}4)
\end{aligned}
$$

$$
\begin{aligned}
h_5 = &\left\{m_3 l_2 l_{g3}\sin\theta_3 + m_3 l_1 l_{g3}\sin(\theta_2 + \theta_3)\right\}\dot{\theta}_1^2 \\
&+ \left(m_3 l_2 l_{g3}\sin\theta_3\right)\dot{\theta}_2^2 \\
&+ \left(2m_3 l_2 l_{g3}\sin\theta_3\right)\dot{\theta}_1\dot{\theta}_2 \quad\quad\cdots(2\text{-}5)
\end{aligned}
$$

The gravity term vector g is expressed by the following Equation (3).

$$
g = \begin{bmatrix} g_1 & g_2 & g_3 & g_4 & g_5 \end{bmatrix}^t \quad\cdots(3)
$$

Each element of the gravity term vector g is calculated by the following equations (3-1) to (3-5).

$$
g_1 = 0 \quad\quad\cdots(3\text{-}1)
$$

$$g_2 = \{m_1 + m_2 + m_3\}g \qquad \cdots(3\text{-}2)$$

$$g_3 = \{(m_1 l_{g1} + m_2 l_1 + m_3 l_1)\sin\theta_1 + (m_2 l_{g2} + m_3 l_2)\sin(\theta_1 + \theta_2) + m_3 l_{g3}\sin(\theta_1 + \theta_2 + \theta_3)\}g \cdots(3\text{-}3)$$

$$g_4 = \{(m_2 l_{g2} + m_3 l_2)\sin(\theta_1 + \theta_2) + m_3 l_{g3}\sin(\theta_1 + \theta_2 + \theta_3)\}g \qquad \cdots(3\text{-}4)$$

$$g_5 = \{m_3 l_{g3}\sin(\theta_1 + \theta_2 + \theta_3)\}g \qquad \cdots(3\text{-}5)$$

The torque coefficient matrix D is expressed by the following Equation (4).

$$D = \begin{bmatrix} 1 & 1 & 0 \\ 1 & 1 & -1 \\ 0 & -1 & 1 \end{bmatrix} \cdots(4)$$

Further, the torque $\tau$ is expressed by the following Equation (5). The value "$\tau_1$" is the torque around the ankle joint. The value "$l_2$" is the torque around the knee joint. The value "$\tau_3$" is the torque around the hip joint. The torque around the ankle joint is the output torque output by the ankle actuator 15. The torque around the knee joint is the output torque output by the knee actuator 14. The torque around the hip joint is the output torque output by the hip actuator 13.

$$\tau = \begin{bmatrix} 0 \\ 0 \\ \tau_1 \\ \tau_2 \\ \tau_3 \end{bmatrix} \cdots(5)$$

The torque $\tau$ is calculated by the following Equation (6). The generalized coordinate X is expressed by Equation (6-1). x is an X coordinate value. z is a Z coordinate value.

$$M(x)\ddot{x} + h(\theta, \dot{\theta}) + g = \tau \quad \cdots(6)$$

$$X = \begin{bmatrix} x & z & \theta_1 & \theta_2 & \theta_3 \end{bmatrix} \cdots(6\text{-}1)$$

Also, let the generalized coordinate X(k) of each joint be the following Equation (7). The value "k" indicates the kth in time series (current value). The value "$x_1$" is the X coordinate of the ankle joint when the forward direction is positive. The value "$x_2$" is the X coordinate of the knee joint when the forward direction is positive. The value "$x_3$" is the X coordinate of the hip joint when the forward direction is positive. The value "zi" is the Z coordinate of the ankle joint when the upward direction is positive. The value "$z_2$" is the Z coordinate of the knee joint when the upward direction is positive. The value "$z_3$" is the Z coordinate of the hip joint when the upward direction is positive.

$$X(k)$$
$$= [x_1(k); z_1(k); \theta_1(k); \theta_2(k); \theta_3(k); \dot{x}_1(k); \dot{z}_1(k); \dot{\theta}_1(k); \dot{\theta}_2(k);$$
$$\dot{\theta}_3(k); x_2(k); z_2(k); x_3(k); z_3(k); \dot{x}_2(k); \dot{z}_2(k); \dot{x}_3(k); \dot{z}_3(k)] \qquad \cdots(7)$$

Then, X (k + 1) is expressed by the following Equation (8). F is the floor reaction force value. H is the Coriolis force h. G is the gravitational term vector g.

$$X(k+1) = X(k) + \Delta t \cdot \dot{X}(k)$$
$$= A \cdot X(k) + \Delta t \cdot (B \cdot U + C)$$

$$= \begin{bmatrix} eye(5) & \Delta t \cdot eye(5) & zeros(5) & zeros(5,3) \\ zeros(5) & eye(5) & zeros(5) & zeros(5,3) \\ zeros(5) & zeros(5) & \begin{bmatrix} 1 & 0 & 0 & 0 & \Delta t \\ 0 & 1 & 0 & 0 & 0 \\ 0 & 0 & 1 & 0 & 0 \\ 0 & 0 & 0 & 1 & 0 \\ 0 & 0 & 0 & 0 & 1 \end{bmatrix} & \begin{bmatrix} 0 & 0 & 0 \\ \Delta t & 0 & 0 \\ 0 & \Delta t & 0 \\ 0 & 0 & \Delta t \\ 0 & 0 & 0 \end{bmatrix} \\ zeros(3,5) & zeros(3,5) & zeros(3,5) & eye(3) \end{bmatrix} \cdot X(k)$$

$$+ \Delta t \cdot \left( \begin{bmatrix} \begin{bmatrix} zeros(5) & zeros(5) \\ zeros(5) & M^{-1} \end{bmatrix} & zeros(10,8) \\ zeros(8,10) & zeros(8) \end{bmatrix} \cdot \begin{bmatrix} zeros(7,1) \\ D \cdot \begin{bmatrix} \tau_1 \\ \tau_2 - F \cdot li \cdot \sin\theta_A \\ \tau_3 - F \cdot li \cdot \cos\theta_A \cdot \sin\theta_K \end{bmatrix} \\ zeros(8,1) \end{bmatrix} + \begin{bmatrix} zeros(5,1) \\ M^{-1}(-H-G) \\ zeros(8,1) \end{bmatrix} \right) \quad \cdots(8)$$

The torque estimation unit 2121 calculates the stance torque by sequentially calculating $\tau$ in Equation (8). By adopting the 3-link model in the sagittal plane in this way, the accuracy can be improved. Since the floor reaction force value F is used in the equations of motion, the stance torque can be calculated in consideration of the impact force from the floor surface. In addition, in order to further improve the accuracy, an equation of motion in the coronal plane may be added as an extension.

The torque estimation unit 2121 calculates the swing torque by the above-mentioned equations of motion (1) to (8). However, when calculating the swing torque, the value "$m_1$" is the mass around the hip joint. The value "$m_2$" is the mass around the knee joint. The value "$m_3$" is the mass around the ankle joint. The value "$l_1$" is the length from the crotch to the knee. The value "$l_2$" is the length from the knee to the waist. The value "$l_3$" is the length from the ankle to the toe.

The value "$l_{g1}$" is the length from the hip joint to the center of gravity of the femur. The value "$lg_2$" is the length from the knee joint to the center of shin. The value "$l_{g3}$" is the length from the ankle joint to the center of gravity of the foot. The value "$I_1$" is the inertia around the hip joint. The value "$I_2$" is the inertia around the knee joint. The value "$I_3$" is the inertia around the ankle joint. The value "$\theta_1$" is the hip joint angle. The value "$\theta_2$" is the knee joint angle. The value "$\theta_3$" is the ankle joint angle. The value "$\tau_1$" is the torque around the hip joint. The value "$l_2$" is the torque around the knee joint. The value "$\tau_3$" is the torque around the ankle joint. The value "$x_1$" is the X coordinate of the hip joint when the forward direction is positive. The value "$x_2$" is the X coordinate of the knee joint when the forward direction is positive. The value "$x_3$" is the X coordinate of the ankle joint when the forward direction is positive. The value "$z_1$" is the Z coordinate of the hip joint when the upward direction is positive. The value "$z_2$" is the Z coordinate of the knee joint when the upward direction is positive. The value "$z_3$" is the Z coordinate of the ankle joint when the upward direction is positive. The torque coefficient matrix D for calculating the swing torque is the following Equation (9).

$$D = \begin{bmatrix} 1 & -1 & 0 \\ 1 & 1 & -1 \\ 0 & 0 & 1 \end{bmatrix} \quad \cdots(9)$$

The torque estimation unit 2121 outputs the calculated stance torque and swing torque of the actuators 13, 14 and 15 of each leg to the torque output smoothing unit 2123.

Subsequently, the stance/swing determination unit 2122 determines whether the state of each leg is the stance state or the swing state on the basis of the load value detected by the foot sole load sensor 18 attached to the leg. For example, when the load value detected by the foot sole load sensor 18 is large (for example, equal to or greater than a first threshold value), the stance/swing determination unit 2122 makes a determination of a stance, and when the load value detected by the foot sole load sensor 18 is small (for example, equal to or less than a second threshold value), the stance/swing determination unit 2122 makes a determination of an swing. The first threshold value is the load value when the sole of the foot is in contact with the ground. The second threshold value is the load value when the sole of the foot is not in contact with the ground.

The stance/swing determination unit 2122 determines the state of the corresponding leg on the basis of the load value detected by the foot sole load sensor 18 of each leg. Therefore, it is possible to accurately make a determination of a stance or a swing for all agile movements such as running without the heel making contact with the ground, one leg movements, and irregular walking movements. For example, the stance/swing determination unit 2122 determines the state of the right leg on the basis of only the value of the load detected by the foot sole load sensor 18 of the right leg, even if a one-leg movement in which only the right leg repeats the swing and the stance. For this reason, the state of the right leg can be accurately determined.

After that, the stance/swing determination unit 2122 decides the drive modes of the actuators 13, 14 and 15 of each leg. The drive modes include a stance mode that outputs stance torque and a swing mode that outputs swing torque.

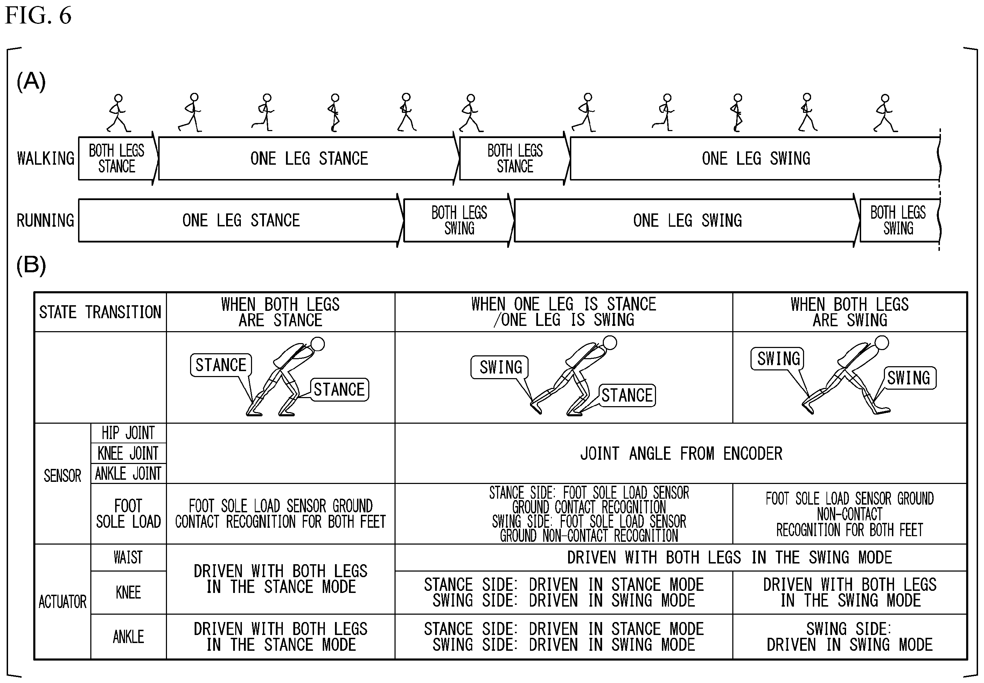

FIG. 6 is a diagram showing the detection result of each sensor and the drive mode of each actuator according to the state of the user.

As shown in FIG. 6A, when the user is walking, the states of both legs stance, and one-leg stance/one-leg swing are repeated in that order. Both legs stance is a state in which both legs are being the stance. One-leg stance/one-leg swing is a state in which one leg is being the stance and the other leg is being the swing. On the other hand, when the user is running, the states of one-leg stance/one-leg swing and both legs swing are repeated in that order. Both legs swing is a state in which both legs are being the swing.

As shown in FIG. 6B, when both legs are being the stance, the joint sensors 23, 24, and 25 detect the joint angle from the encoder, the foot sole load sensor 18 detects ground contact (equal to or greater than the first threshold value) at both feet, and each of the actuators 13, 14 and 15 of both legs is driven in the stance mode.

In addition, the following processing is performed during a one-leg stance/one-leg swing period. That is, each of the joint sensors 23, 24, and 25 detects the joint angle from the encoder, the foot sole load sensor 18 on the stance side detects ground contact, and the foot sole load sensor 18 on the swing side detects non-ground contact (equal to or less than the second threshold). The hip actuators 13 of both legs are driven in the swing mode, the knee actuator 14 on the stance side is driven in the stance mode, and the knee actuator 14 on the swing side is driven in the swing mode. The ankle actuator 15 on the stance side is driven in the stance mode, and the ankle actuator 15 on the swing side is driven in the swing mode.

Further, when both legs of the user are being the swing, the joint sensors 23, 24, 25 detect the joint angle from the encoder, the foot sole load sensor 18 detects that both legs are not touching the ground, and the actuators 13, 14, 15 of both legs are driven in the swing mode.

However, the control model during a stance period and the control model during a swing period differ. Therefore, when the drive modes of the actuators 13, 14 and 15 are switched only based on whether or not the sole of the user's foot is in contact with the ground, at the moment of switching from the stance mode to the swing mode or the moment of switching from the swing mode to the stance mode, non-linear torque is output from the actuators 13, 14 and 15. For this reason, the difference in output torque at the time of switching is applied to the body as an excessive load, and the user feels uncomfortable as compared with the normal time, making movement difficult.

Therefore, the stance/swing determination unit 2122 measures the elapsed time t after the drive mode is switched. Then, the stance/swing determination unit 2122 outputs the drive mode and the elapsed time t of the actuators 13, 14 and 15 of each leg to the torque output smoothing unit 2123.

The torque output smoothing unit 2123 computes the output torque of the actuators 13, 14, 15 of each leg on the basis of the drive mode and elapsed time t determined by the stance/swing determination unit 2122 and the stance torque and swing torque calculated by the torque estimation unit 2121. Specifically, the torque output smoothing unit 2123 executes a smoothing process so that the transition of the torque value becomes smooth when the drive modes of the actuators 13, 14 and 15 switch. That is, the torque output smoothing unit 2123 calculates the torque so that the transition of the torque value becomes gentle when the drive mode is switched.

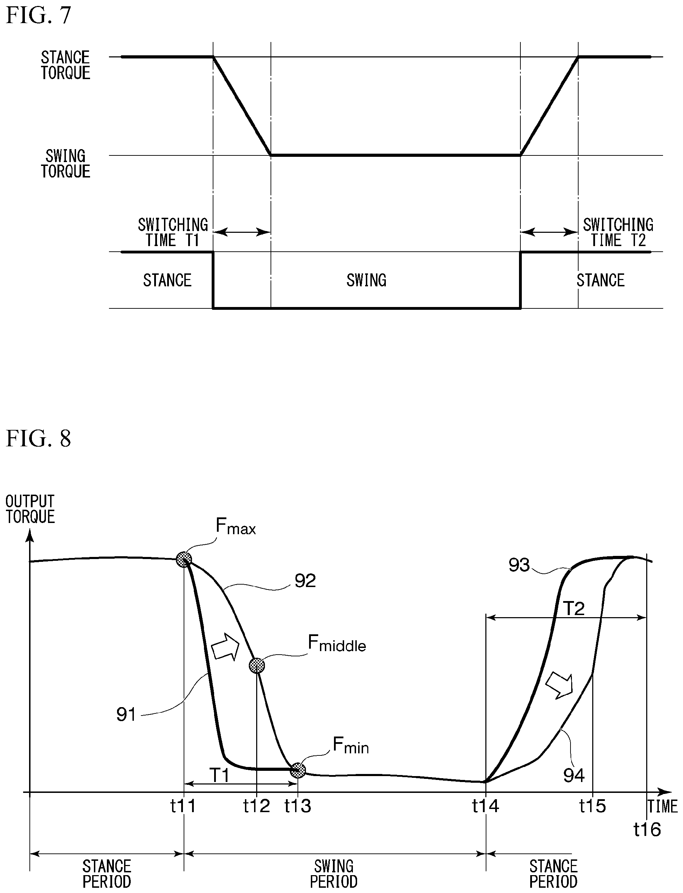

FIG. 7 is a graph showing an outline of an exemplary smoothing process.

The torque output smoothing unit 2123 provides a switching time T1 from the stance mode to the swing mode. The torque output smoothing unit 2123 sets output torque $\tau$ so as to smoothly transition from the stance torque $\tau_s$ to the swing torque $\tau_f$ during the switching time T1 (that is, the elapsed time $t \leq$ the switching time T1) after the drive mode is switched from the stance mode to the swing mode. More specifically, the output torque $\tau$ is calculated by the following Equation (10).

$$\tau = \frac{t}{T1} \times \tau_f + \left(1 - \frac{t}{T1}\right) \times \tau_s \qquad \cdots (10)$$

Further, the torque output smoothing unit 2123 provides a switching time T2 from the swing mode to the stance mode. The torque output smoothing unit 2123 sets the output torque $\tau$ so as to smoothly transition from the swing torque $\tau_f$ to the swing torque $\tau_s$ during the switching time T2 (that is, the elapsed time $t \leq$ the switching time T2) after switching from the swing mode to the stance mode. More specifically, the output torque $\tau$ is calculated by the following Equation (11).

$$\tau = \frac{t}{T2} \times \tau_s + \left(1 - \frac{t}{T2}\right) \times \tau_f \qquad \cdots (11)$$

In this way, the torque output smoothing unit 2123 calculates the stance torque and the swing torque according to the ratio of the elapsed time t to the switching times T1 and T2. The torque output smoothing unit 2123 calculates the output torque $\tau$ by adding the stance torque and the swing torque in accordance with the ratio.

Another example of the smoothing process executed by the torque output smoothing unit 2123 will be described.

FIG. 8 is a graph showing the transition of the output torque of the actuator according to the present invention.

The horizontal axis of the graph shown in this figure is time, and the vertical axis is output torque.

The process shown in this figure differs from the smoothing process shown in FIG. 7 in that parameters $\alpha 1$, $\beta 1$, $\alpha 2$, $\beta 2$ used in the smoothing process are changed according to the rate of change of the output torque $\tau$ due to the smoothing process.

Specifically, first, the torque output smoothing unit 2123 sets "$F_{max} = \tau_s$" and "$F_{middle} = F_{max}/2$" when the drive mode is switched from the stance mode to the swing mode at time t11. Then, the torque output smoothing unit 2123 calculates the output torque $\tau$ by the following Equation (12) during the period until time t12 when the output torque $\tau$ becomes "$F_{middle}$". t is the elapsed time from time t11. The switching time T1 is set in advance, and in this example is the period from time t11 to time t13. The parameter $\alpha 1$ and parameter $\beta 1$ are parameters including time-dependent mathematical expressions.

$$\tau = \frac{t}{T1} \cdot \tau_f \cdot \alpha 1 + \left(1 - \frac{t}{T1}\right) \cdot \tau_s \cdot \beta 1 \qquad \cdots (12)$$

Subsequently, the torque output smoothing unit 2123 resets the parameter $\alpha 1$ and the parameter $\beta 1$ so that the rate of change of the output torque $\tau$ becomes steeper or smoother according to the rate of change of the output torque $\tau$ during the period from the time t11 to the time t12. Then, the torque output smoothing unit 2123 calculates the output torque $\tau$ by the above Equation (12) using the parameter $\alpha 1$ and the parameter $\beta 1$ that were reset during the period from time t12 to time t13. Thereby, the output torque $\tau$ at T13, the end of the switching time T1, is the swing torque ($F_{min} = \tau_f$).

The solid line 91 shows the transition of the swing torque before the smoothing process at the switching time T1. On the other hand, the solid line 92 shows the transition of the output torque after the smoothing process at the switching time T1. By resetting the parameter $\alpha 1$ and the parameter $\beta 1$ during the smoothing process in this way, it is possible to smooth the change in the output torque $\tau$ more stably than in the process shown in FIG. 7. Thereby, it is possible to prevent the output torque from suddenly changing when the drive mode is switched, and to reduce the discomfort felt by the user.

Further, the torque output smoothing unit 2123 calculates the output torque $\tau$ by the following Equation (13) during the period until time t15 when the output torque $\tau$ becomes "$F_{middle}$" when the drive mode is switched from the swing mode to the stance mode at time t14. t is the elapsed time from time t14. The switching time T2 is set in advance, and in this example is the period from time t14 to time t16. The parameter $\alpha 2$ and parameter $\beta 2$ are parameters including time-dependent mathematical expressions.

$$\tau = \frac{t}{T2} \cdot \tau_s \cdot \alpha 2 + \left(1 - \frac{t}{T2}\right) \cdot \tau_f \cdot \beta 2 \qquad \cdots (13)$$

Subsequently, the torque output smoothing unit 2123 resets the parameter $\alpha 2$ and the parameter $\beta 2$ so that the rate of change of the output torque $\tau$ becomes steeper or smoother according to the rate of change of the output torque $\tau$ during the period from the time t14 to the time t15. Then, the torque output smoothing unit 2123 calculates the output torque $\tau$ by the above Equation (13) using the parameter $\alpha 2$ and the parameter $\beta 2$ that were re-set, during the period from time t15 to time t16.

The solid line 93 shows the transition of the stance torque before the smoothing process at the switching time T2. On the other hand, the solid line 94 shows the transition of the output torque after the smoothing process at the switching time T2. By resetting the parameter $\alpha2$ and the parameter $\beta2$ during the smoothing process in this way, it is possible to smooth the change in the output torque $\tau$ more stably than in the process shown in FIG. 7. Thereby, it is possible to prevent the output torque from suddenly changing when the drive mode is switched, and to reduce the discomfort felt by the user.

Note that the torque output smoothing unit 2123 may adaptively change the switching time T1 and the switching time T2 by machine learning. For example, the torque output smoothing unit 2123 may change the time from when the drive mode is switched until the load value detected by the foot sole load sensor 18 reaches the maximum value or the minimum value, as the switching time. This makes it possible to optimally smooth the output torque at the time of switching.

The torque output smoothing unit 2123 outputs the calculated output torque of each actuator 13, 14 and 15 of each leg to the actuator control unit 213.

The actuator control unit 213 controls the rotation angles of the actuators 13, 14 and 15 with an angle controller $K_{ci}(s)$ on the basis of the output torque. "s" indicates the frequency domain of the control system. Subsequently, the actuator control unit 213 causes the actuators 13, 14 and 15 of each leg to output the torque $\tau$ with a force controller $K_{bi}(s)$.

Thereby, the interaction force between suits and person $D_k$ applied by the user, the applied torque $I_k$ applied by the user, and the output torque $\tau$ in the kth of the time series (current value) become the dynamics $P(s)$ of each actuator. Each joint sensor 23, 24, 25 detects each joint angle $\theta_k$ in the kth of the time series according to the dynamics $G(s)$ of the powered suit 100 based on the dynamics $P(s)$ of the actuators 13, 14, 15 and outputs the detected each joint angle $\theta_k$ to the torque estimation unit 2121. Then, the control device 21 repeats the above-described processing.

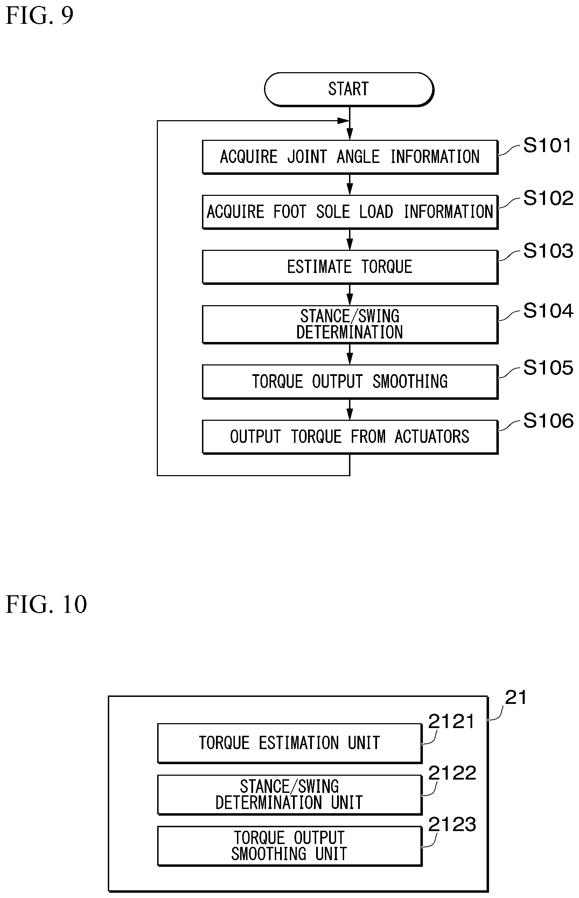

FIG. 9 is a flowchart showing the processing of the powered suit.

First, the user puts on the powered suit 100. Since each foot sole load sensor 18 is attached between the insole of the foot harness 17 and the shoe sole plate 16, when the user wears the foot harnesses 17, the foot sole load sensors 18 can measure the weight applied from the soles of the user.

The user operates a power button of the control device 21 provided in the powered suit 100 to turn on the power. As a result, the control device 21 is started. The user walks while wearing the powered suit 100. The user may load luggage on the loading platform 20 of the powered suit 100 and walk. The actuator control unit 213 of the control device 21 controls the hip actuator 13, the knee actuator 14, and the ankle actuator 15 so as to reduce the load on the user due to the weight of the luggage and the powered suit 100. Thereby, the powered suit 100 tracks various motions of the user.

While the control device 21 is being driven, the information acquisition unit 211 acquires joint angle information from the joint sensors 23, 24, and 25 at predetermined intervals (Step S101). Further, while the control device 21 is being driven, the information acquisition unit 211 acquires the sole load information from each foot sole load sensor 18 at a predetermined interval (Step S102). The predetermined interval is, for example, every short time such as every 10 milliseconds.

The torque estimation unit 2121 calculates the stance torque and swing torque of each actuator 13, 14, 15 of each leg based on the joint angle information and the sole load information acquired by the information acquisition unit 211, and estimates the torque (Step S103). The stance/swing determination unit 2122 determines the drive modes of the actuators 13, 14 and 15 of each leg on the basis of the sole load information acquired by the information acquisition unit 211. The stance/swing determination unit 2122 measures the elapsed time after the drive mode is switched (Step S104).

The torque output smoothing unit 2123 smooths the output torque on the basis of the drive mode and elapsed time determined by the stance/swing determination unit 2122 and the stance torque and swing torque calculated by the torque estimation unit 2121 (Step S105). The actuator control unit 213 causes the actuators 13, 14 and 15 of each leg to output the torque (Step S106). After that, the process returns to the process of Step S101, and the control device 21 repeats the process from steps S101 to S106 until the process is completed.

According to the above processing, the accurate torque required for each joint to support the load is calculated, and the actuators 13, 14 and 15 can output that torque. For this reason, even if there is a large fluctuation from low response to high response in all movement patterns of the user, such as slow walking movements and agile movements of running, it is always possible to follows the user's movements and it is possible to realize assist for load reduction in a timely and appropriate manner at each movement. For example, the torque output smoothing unit 2123 smooths the output torque when switching between the stance and the swing. As a result, it is possible to prevent the output torque from suddenly changing, and so a sense of discomfort during motion is alleviated, and it is possible to realize an improvement in the ability to track movements of the user.

Further, even during an swing period, the torque estimation unit 2121 calculates the swing torque and causes the actuators 13, 14 and 15 to output the swing torque, thereby assisting the load of the powered suit 100 itself and enabling smooth movement tracking without hindering the user's motion. Therefore, agile motion is possible in the state of the powered suit 100 being worn.

Although one embodiment of the present invention has been described above, the present invention is not limited to the above embodiment, and various modifications can be made without departing from the scope of the present invention.

For example, the foot sole load sensor 18 may be inserted inside the foot harness 17 during use by the user. The foot sole load sensor 18 may be provided on a side of the ground contact surface of the foot harness 17.

In the above description, it was shown that the foot sole load sensor 18 has an area inside the foot harness 17 so as to cover the entire surface of the sole. However, the foot sole load sensor 18 should be capable of measuring the load applied to the ground contact surface from the shoe sole plate 16 or the foot harness 17 even when the position where that load is applied deviates.

The above description illustrated a case of controlling the powered suit 100 but is not limited thereto, and the control device 21 can be applied to general control of a multi-joint robot or the like (for example, a humanoid robot) having a non-linear mode transition.

Further, in the above description, the powered suit 100 is provided with the hip actuator 13, the knee actuator 14, and the ankle actuator 15 corresponding respectively to each joint, but is not limited thereto. The powered suit 100 may be provided with at least one of the actuators 13, 14, and 15. For example, the powered suit 100 may be provided with only the hip actuator 13 and the knee actuator 14, and does not have to be provided with the ankle actuator 15. Alternatively, the ankle actuator 15 may be an actuator that does not use a control signal, such as a mechanical leaf spring. In this case, the ankle joint sensor 25 is an angle detection sensor that detects the bending angle of the leaf spring or a force sensor that detects the reaction force of the leaf spring. The torque estimation unit 2121 calculates the torque around the hip joint and the torque around the knee joint by Equation (6) on the basis of the bending angle of the leaf spring or the reaction force of the leaf spring detected by the ankle joint sensor 25.

FIG. 10 is a diagram showing the minimum configuration of the control device.

As one aspect of the load reduction device, the control device 21 may have at least the functions of the torque estimation unit 2121, the stance/swing determination unit 2122, and the torque output smoothing unit 2123 described above.

The torque estimation unit 2121 calculates the stance torque output by the actuators 13, 14 and 15 during a stance period and the swing torque output by the actuators 13, 14 and 15 during a swing period on the basis of the load values applied to the soles of the feet and the angle of each joint of the leg. The actuators 13, 14 and 15 output torque to reduce the load on the user at the joints of the legs of the user.

The stance/swing determination unit 2122 determines whether the leg state is the stance state or the swing state.

The torque output smoothing unit 2123 smooths the transition of the torque output by each of the actuators 13, 14 and 15 according to the elapsed time based on the stance torque and the swing torque when the leg state is switched. That is, when the state of each leg is switched, the torque output smoothing unit 2123 calculates the torque whose transition was smoothed according to the elapsed time.

The above-mentioned control device may also be a computer provided with hardware such as the CPU (Central Processing Unit) 101, the ROM (Read Only Memory) 102, the RAM (Random Access Memory) 103, an HDD (Hard Disk Drive) 104, and the wireless communication device 105.

The control device described above has a computer system inside. The process of each processing described above is stored in a computer-readable recording medium in the form of a program, with the process being performed by the computer reading and executing this program. Here, the computer-readable recording medium refers to a magnetic disk, a magneto-optical disk, a CD-ROM, a DVD-ROM, a semiconductor memory, or the like. This computer program may be distributed to a computer via a communication line, and the computer receiving the distribution may execute the program.

Further, the above-mentioned program may be for realizing some of the functions described above.

Moreover, the above-mentioned program may be a so-called differential file (differential program) that can realize the above-mentioned functions in combination with a program already recorded in the computer system.

Priority is claimed on Japanese Patent Application No. 2018-219220, filed November 22, 2018, the content of which is incorporated herein by reference.

INDUSTRIAL APPLICABILITY

According to the present invention, it is possible to provide smooth load reduction with respect to all motions of a user.

[Reference Signs List]

100: Powered suit
11: Skeleton portion
12: Belt
13: Hip actuator
14: Knee actuator

15: Ankle actuator
16: Shoe sole plate
17: Foot harness
18: Foot sole load sensor
20: Loading platform
21: Control device
22: Battery
23: Hip joint sensor
24: Knee joint sensor
25: Ankle joint sensor
211: Information acquisition unit
212: Integrated control unit
2121: Torque estimation unit
2122: Stance/swing determination unit
2123: Torque output smoothing unit
213: Actuator control unit
214: Power supply unit

**Claims**

1. A load reduction device (21) comprising:

    an information acquisition means (211) for acquiring, at predetermined intervals, loads on soles of the legs of a user and an angle at each of joints of the legs of the user;
    a torque estimation means (2121) for calculating sequentially stance torque output during a stance period and swing torque output during a swing period by a drive mechanism for outputting torque to reduce a load on the user at the joints of the legs, on the basis of values of the loads on the soles of the legs and the angle at each of the joints of the legs that are acquired at the predetermined intervals;
    a stance/swing determination means (2122) for determining whether each of the legs is in a stance state or a swing state; and
    a torque output smoothing means (2123) for smoothing, upon a switch in a state of the each of legs, a transition in the torque output by the drive mechanism in a switching time T1, in accordance with an elapsed time t on the basis of the stance torque $\tau_s$ and the swing torque $\tau_f$ that are calculated sequentially,
    wherein the torque output smoothing means (2123) is configured for providing the switching time T1 for switching between a period in which the drive mechanism outputs the stance torque $\tau_s$ and a period in which the drive mechanism outputs the swing torque $\tau_f$, the switching time T1 including a first period of time until the torque to be output reaches a predetermined torque indicating half of the stance torque $\tau_s$, and a second period of time after the torque to be output reached the predetermined torque, calculating the torque $\tau$ for the first period according to an equation

$$\tau = \frac{t}{T1} \cdot \tau_f \cdot \alpha 1 + \left(1 - \frac{t}{T1}\right) \cdot \tau_s \cdot \beta 1$$

,

    a value $\alpha 1$ indicating a first parameter, a value $\beta 1$ indicating a second parameter, a value t indicating the elapsed time in the switching time T1, a value $\tau_s$ indicating a torque at a beginning of the switching time T1, and a value $\tau_f$ indicating a torque at an end of the switching time T1,
    resetting the first parameter $\alpha 1$ and the second parameter $\beta 1$, according to a rate of change of the torque in the first period calculated by the equation, and
    calculating the torque $\tau$ for the second period according to the equation using the re-set first parameter $\alpha 1$ and the re-set second parameter $\beta 1$.

2. The load reduction device (21) according to claim 1, wherein
    the torque output smoothing means (2123) calculates the torque in the first period obtained by adding the stance torque $\tau_s$ and the swing torque $\tau_f$ according to the first parameter $\alpha 1$ and the second parameter $\beta 1$ for the smoothing

and the ratio of the elapsed time t to the switching time T1, resets the first parameter $\alpha 1$ and the second parameter $\beta 1$ used for the smoothing in the second period according to the rate of change of the calculated torque in the first period to calculate the torque in the second period using the reset first parameter $\alpha 1$ and the reset second parameter $\beta 1$.

3. The load reduction device (21) according to claim 1 or claim 2, wherein
   the torque estimation means (2121) calculates the stance torque $\tau_s$ and the swing torque $\tau_f$ of each joint on the basis of a control model in which each joint on the sagittal plane of the user is linked.

4. The load reduction device (21) according to any one of claim 1 to claim 3, wherein
   the stance/swing determination means (2122) determines whether the state of each leg is in the stance state or the swing state on the basis of the value of the loads on the soles of the legs.

5. A load reduction method comprising:

   acquiring, at predetermined intervals, loads on soles of the legs of a user and an angle at each of joints of the legs of the user;
   calculating sequentially stance torque output during a stance period and swing torque output during a swing period by a drive mechanism for outputting torque to reduce a load on the user at the joints of the legs, on the basis of values of the loads on the soles of the legs and the angle at each of the joints of the legs that are acquired at the predetermined intervals;
   determining whether each of the legs is in a stance state or a swing state; and
   upon a switch in a state of the each of legs, smoothing a transition in the torque output by the drive mechanism in a switching time T1, in accordance with an elapsed time t on the basis of the stance torque $\tau_s$ and the swing torque $\tau_f$ that are calculated sequentially,
   wherein the smoothing includes:
   providing the switching time T1 for switching between a period in which the drive mechanism outputs the stance torque $\tau_s$ and a period in which the drive mechanism outputs the swing torque $\tau_f$, the switching time T1 including a first period of time until the torque to be output reaches a predetermined torque indicating half of the stance torque $\tau_s$, and a second period of time after the torque to be output reached the predetermined torque;
   calculating the torque $\tau$ for the first period according to an equation

$$\tau = \frac{t}{T1} \cdot \tau_f \cdot \alpha 1 + \left(1 - \frac{t}{T1}\right) \cdot \tau_s \cdot \beta 1$$

   ,

   a value $\alpha 1$ indicating a first parameter, a value $\beta 1$ indicating a second parameter, a value t indicating the elapsed time in the switching time T1, a value $\tau_s$ indicating a torque at a beginning of the switching time T1, and a value $\tau_f$ indicating a torque at an end of the switching time T1;
   resetting the first parameter $\alpha 1$ and the second parameter $\beta 1$, according to a rate of change of the torque in the first period calculated by the equation, ; and
   calculating the torque $\tau$ for the second period according to the equation using the re-set first parameter $\alpha 1$ and the re-set second parameter $\beta 1$.

6. A program that causes a computer of a load reduction device to execute processes, the processes comprising:

   acquiring, at predetermined intervals, loads on soles of the legs of a user and an angle at each of joints of the legs of the user;
   calculating sequentially stance torque output during a stance period and swing torque output during a swing period by a drive mechanism for outputting torque to reduce a load on the user at the joints of the legs, on the basis of values of the loads on the soles of the legs and the angle at each of the joints of the legs that are acquired at the predetermined intervals;
   determining whether each of the legs is in a stance state or a swing state; and
   upon a switch in a state of the each of legs, smoothing a transition in the torque output by the drive mechanism in a switching time T1, in accordance with an elapsed time t on the basis of the stance torque $\tau_s$ and the swing torque $\tau_f$ that are calculated sequentially,
   wherein the smoothing includes:

providing the switching time T1 for switching between a period in which the drive mechanism outputs the stance torque $\tau_s$ and a period in which the drive mechanism outputs the swing torque $\tau_f$,

the switching time T1 including a first period of time until the torque to be output reaches a predetermined torque indicating half of the stance torque $\tau_s$, and a second period of time after the torque to be output reached the predetermined torque;

calculating the torque $\tau$ for the first period according to an equation

$$\tau = \frac{t}{T1} \cdot \tau_f \cdot \alpha 1 + \left(1 - \frac{t}{T1}\right) \cdot \tau_s \cdot \beta 1 \quad ,$$

a value $\alpha 1$ indicating a first parameter, a value $\beta 1$ indicating a second parameter, a value t indicating the elapsed time in the switching time T1, a value $\tau_s$ indicating a torque at a beginning of the switching time T1, and a value $\tau_f$ indicating a torque at an end of the switching time T1;

resetting the first parameter $\alpha 1$ and the second parameter $\beta 1$, according to a rate of change of the torque in the first period calculated by the equation, ; and

calculating the torque $\tau$ for the second period according to the equation using the re-set first parameter $\alpha 1$ and the re-set second parameter $\beta 1$.

## Patentansprüche

1. Lastreduzierungsvorrichtung (21), die aufweist:

eine Informationserlangungsvorrichtung (211) zur Erlangung von Lasten auf Sohlen der Beine eines Benutzers und von Winkeln an jedem der Gelenke der Beine des Benutzers in vorgegebenen Zeitabständen;

eine Drehmomentschätzeinrichtung (2121), um nacheinander das Standdrehmoment während einer Standzeitspanne und das Schwungdrehmoment, das während einer Schwungzeitspanne durch einen Antriebsmechanismus zur Ausgabe von Drehmoment ausgegeben wird, um eine Last auf den Benutzer an den Gelenken der Beine zu verringern, auf der Basis von Werten der Lasten auf den Sohlen der Beine und der Winkel an jedem der Gelenke der Beine, die während den vorgegebenen Zeitabständen erfasst werden, zu berechnen;

eine Stand-/Schwungbestimmungseinrichtung (2122), um zu bestimmen, ob jedes der Beine in einem Standzustand oder einem Schwungzustand ist; und

eine Drehmomentausgabe-Glättungseinrichtung (2123), um bei einem Wechsel in einem Zustand jedes der Beine einen Übergang des Drehmoments, das durch den Antriebsmechanismus in einer Umschaltzeit T1 ausgegeben wird, gemäß einer vergangenen Zeit t auf der Basis des Standdrehmoments $\tau_s$ und des Schwungdrehmoments $\tau_f$, die nacheinander berechnet werden, zu glätten,

wobei die Drehmomentausgabe-Glättungseinrichtung (2123) konfiguriert ist, um die Umschaltzeit T1 zum Umschalten zwischen einer Zeitspanne, in welcher der Antriebsmechanismus das Standdrehmoment $\tau_s$ ausgibt, und einer Zeitspanne, in welcher der Antriebsmechanismus das Schwungdrehmoment $\tau_f$ ausgibt, bereitzustellen, wobei die Umschaltzeit T1 eine erste Zeitspanne, bis das Drehmoment, das ausgegeben werden soll, ein vorgegebenes Drehmoment, das die Hälfte des Standdrehmoments $\tau_s$ anzeigt, erreicht, und eine zweite Zeitspanne, nachdem das Drehmoment, das ausgegeben werden soll, das vorgegebene Drehmoment erreicht hat, umfasst,

um das Drehmoment $\tau$ für die erste Zeitspanne gemäß folgender Gleichung zu berechnen:

$$\tau = \frac{t}{T1} \cdot \tau_f \cdot \alpha 1 + \left(1 - \frac{t}{T1}\right) \cdot \tau_s \cdot \beta 1$$

wobei ein Wert $\alpha 1$ einen ersten Parameter anzeigt, ein Wert $\beta 1$ einen zweiten Parameter anzeigt, ein Wert t die vergangene Zeit in der Umschaltzeit T1 anzeigt, ein Wert $\tau_s$ ein Drehmoment am Anfang der Umschaltzeit T1 anzeigt und ein Wert $\tau_f$ ein Drehmoment an einem Ende der Umschaltzeit T1 anzeigt, um den ersten Parameter $\alpha 1$ und den zweiten Parameter $\beta 1$ gemäß einer durch die Gleichung berechneten Änderungsgeschwindigkeit des Drehmoments in der ersten Zeitspanne zurückzusetzen, und das Drehmoment $\tau$ für die zweite Zeitspanne gemäß der Gleichung unter Verwendung des zurückgesetzten ersten Parameters α1 und des zurückgesetzten zweiten Parameters β1 zu berechnen.

2. Lastreduzierungsvorrichtung (21) nach Anspruch 1, wobei
die Drehmomentausgabe-Glättungseinrichtung (2123) das Drehmoment in der ersten Zeitspanne, das durch Addieren des Standdrehmoments $\tau_s$ und des Schwungdrehmoments $\tau_f$ erhalten wird, gemäß dem ersten Parameter α1 und dem zweiten Parameter β1 für die Glättung und dem Verhältnis der vergangenen Zeit t zu der Umschaltzeit T1 berechnet, den ersten Parameter α1 und den zweiten Parameter β1, die für das Glätten in der zweiten Zeitspanne verwendet werden, gemäß der Änderungsgeschwindigkeit des berechneten Drehmoments in der ersten Zeitspanne zurücksetzt, um das Drehmoment in der zweiten Zeitspanne unter Verwendung des zurückgesetzten ersten Parameters α1 und des zurückgesetzten zweiten Parameters β1 zu berechnen.

3. Lastreduzierungsvorrichtung (21) nach Anspruch 1 oder Anspruch 2, wobei
die Drehmomentschätzeinrichtung (2121) das Standrehmoment $\tau_s$ und das Schwungdrehmoment $\tau_f$ jedes Gelenks auf der Basis eines Steuermodells, in dem jedes Gelenk auf der Sagittalebene des Benutzers verknüpft ist, berechnet.

4. Lastreduzierungsvorrichtung (21) nach einem der Ansprüche 1 bis 3, wobei
der Stand-/Schwungbestimmungsmechanismus (2122) auf der Basis des Werts der Lasten auf den Sohlen der Beine bestimmt, ob der Zustand jedes Beins in dem Standzustand oder dem Schwungzustand ist.

5. Lastreduzierungsverfahren, das aufweist:

Erlangen von Lasten auf Sohlen der Beine eines Benutzers und von Winkeln an jedem der Gelenke der Beine des Benutzers in vorgegebenen Zeitabständen;
nacheinander Berechnen des Standdrehmoments, das während einer Standzeitspanne ausgegeben wird, und des Schwungdrehmoments, das während einer Schwungzeitspanne durch einen Antriebsmechanismus zur Ausgabe von Drehmoment ausgegeben wird, um eine Last auf den Benutzer an den Gelenken der Beine zu verringern, auf der Basis von Werten der Lasten auf den Sohlen der Beine und der Winkel an jedem der Gelenke der Beine, die während den vorgegebenen Zeitabständen erfasst werden;
Bestimmen, ob jedes der Beine in einem Standzustand oder einem Schwungzustand ist; und
bei einem Wechsel in einem Zustand jedes der Beine Glätten eines Übergangs des Drehmoments, das durch den Antriebsmechanismus in einer Umschaltzeit T1 ausgegeben wird, gemäß einer vergangenen Zeit t auf der Basis des Standdrehmoments $\tau_s$ und des Schwungdrehmoments $\tau_f$, die nacheinander berechnet werden,
wobei das Glätten aufweist:

Bereitstellen der Umschaltzeit T1 zum Umschalten zwischen einer Zeitspanne, in welcher der Antriebsmechanismus das Standdrehmoment $\tau_s$ ausgibt, und einer Zeitspanne, in welcher der Antriebsmechanismus das Schwungdrehmoment $\tau_f$ ausgibt, wobei die Umschaltzeit T1 eine erste Zeitspanne, bis das Drehmoment, das ausgegeben werden soll, ein vorgegebenes Drehmoment, das die Hälfte des Standdrehmoments $\tau_s$ anzeigt, erreicht, und eine zweite Zeitspanne, nachdem das Drehmoment, das ausgegeben werden soll, das vorgegebene Drehmoment erreicht hat, umfasst,
Berechnen des Drehmoments $\tau$ für die erste Zeitspanne gemäß folgender Gleichung:

$$\tau = \frac{t}{T1} \cdot \tau_f \cdot \alpha 1 + (1 - \frac{t}{T1}) \cdot \tau_s \cdot \beta 1$$

wobei ein Wert α1 einen ersten Parameter anzeigt, ein Wert β1 einen zweiten Parameter anzeigt, ein Wert t die vergangene Zeit in der Umschaltzeit T1 anzeigt, ein Wert $\tau_s$ ein Drehmoment am Anfang der Umschaltzeit T1 anzeigt und ein Wert $\tau_f$ ein Drehmoment an einem Ende der Umschaltzeit T1 anzeigt,
Zurücksetzen des ersten Parameters α1 und des zweiten Parameters β1 gemäß einer durch die Gleichung berechneten Änderungsgeschwindigkeit des Drehmoments in der ersten Zeitspanne; und
Berechnen des Drehmoments $\tau$ für die zweite Zeitspanne gemäß der Gleichung unter Verwendung des zurückgesetzten ersten Parameters α1 und des zurückgesetzten zweiten Parameters β1.

6. Programm, das einen Computer einer Lastreduzierungsvorrichtung veranlasst, Verfahren auszuführen, wobei die Verfahren aufweisen:

Erlangen von Lasten auf Sohlen der Beine eines Benutzers und von Winkeln an jedem der Gelenke der Beine des Benutzers in vorgegebenen Zeitabständen;

nacheinander Berechnen des Standdrehmoments, das während einer Standzeitspanne ausgegeben wird, und des Schwungdrehmoments, das während einer Schwungzeitspanne durch einen Antriebsmechanismus zur Ausgabe von Drehmoment ausgegeben wird, um eine Last auf den Benutzer an den Gelenken der Beine zu verringern, auf der Basis von Werten der Lasten auf den Sohlen der Beine und der Winkel an jedem der Gelenke der Beine, die während den vorgegebenen Zeitabständen erfasst werden;

Bestimmen, ob jedes der Beine in einem Standzustand oder einem Schwungzustand ist; und

bei einem Wechsel in einem Zustand jedes der Beine Glätten eines Übergangs des Drehmoments, das durch den Antriebsmechanismus in einer Umschaltzeit T1 ausgegeben wird, gemäß einer vergangenen Zeit t auf der Basis des Standdrehmoments $\tau_s$ und des Schwungdrehmoments $\tau_f$, die nacheinander berechnet werden,

wobei das Glätten aufweist:

Bereitstellen der Umschaltzeit T1 zum Umschalten zwischen einer Zeitspanne, in welcher der Antriebsmechanismus das Standdrehmoment $\tau_s$ ausgibt, und einer Zeitspanne, in welcher der Antriebsmechanismus das Schwungdrehmoment $\tau_f$ ausgibt, wobei die Umschaltzeit T1 eine erste Zeitspanne, bis das Drehmoment, das ausgegeben werden soll, ein vorgegebenes Drehmoment, das die Hälfte des Standdrehmoments $\tau_s$ anzeigt, erreicht, und eine zweite Zeitspanne, nachdem das Drehmoment, das ausgegeben werden soll, das vorgegebene Drehmoment erreicht hat, umfasst,

Berechnen des Drehmoments $\tau$ für die erste Zeitspanne gemäß folgender Gleichung:

$$\tau = \frac{t}{T1} \cdot \tau_f \cdot \alpha1 + (1 - \frac{t}{T1}) \cdot \tau_s \cdot \beta1$$

wobei ein Wert $\alpha1$ einen ersten Parameter anzeigt, ein Wert $\beta1$ einen zweiten Parameter anzeigt, ein Wert t die vergangene Zeit in der Umschaltzeit T1 anzeigt, ein Wert $\tau_s$ ein Drehmoment am Anfang der Umschaltzeit T1 anzeigt und ein Wert $\tau_f$ ein Drehmoment an einem Ende der Umschaltzeit T1 anzeigt,

Zurücksetzen des ersten Parameters $\alpha1$ und des zweiten Parameters $\beta1$ gemäß einer durch die Gleichung berechneten Änderungsgeschwindigkeit des Drehmoments in der ersten Zeitspanne; und

Berechnen des Drehmoments $\tau$ für die zweite Zeitspanne gemäß der Gleichung unter Verwendung des zurückgesetzten ersten Parameters $\alpha1$ und des zurückgesetzten zweiten Parameters $\beta1$.

## Revendications

1. Dispositif de réduction de charge (21) comprenant :

un moyen d'acquisition d'informations (211) pour acquérir, à des intervalles prédéterminés, des charges exercées sur les plantes de pied des jambes d'un utilisateur et des angles au niveau de chacune des articulations des jambes de l'utilisateur;

un moyen d'estimation de couple (2121) pour calculer séquentiellement un couple d'appui délivré en sortie pendant une période d'appui et un couple de balancement délivré pendant une période de balancement au moyen d'un mécanisme d'entraînement pour délivrer en sortie un couple afin de réduire une charge exercée sur l'utilisateur au niveau des articulations des jambes, sur la base de valeurs des charges exercées sur les plantes de pied des jambes et des angles au niveau de chacune des articulations des jambes qui sont acquises aux intervalles prédéterminés ;

un moyen de détermination d'appui/de balancement (2122) pour déterminer si chacune des jambes est dans un état d'appui ou un état de balancement ; et

un moyen de lissage de sortie de couple (2123) pour lisser, lors d'une commutation dans un état de chacune des jambes, une transition dans le couple délivré en sortie par le mécanisme d'entraînement dans un temps de commutation T1, conformément à un temps écoulé t sur la base du couple d'appui $\tau_s$ et du couple de balancement $\tau_f$ qui sont calculés séquentiellement,

dans lequel le moyen de lissage de sortie de couple (2123) est configuré pour fournir le temps de commutation T1 pour commuter entre une période au cours de laquelle le mécanisme d'entraînement délivre en sortie le couple d'appui $\tau_s$ et une période au cours de laquelle le mécanisme d'entraînement délivre en sortie le couple de balancement $\tau_f$, le temps de commutation T1 comportant une première période de temps jusqu'à ce que le couple à délivrer en sortie atteigne un couple prédéterminé indiquant la moitié du couple d'appui $\tau_s$, et une deuxième période de temps après que le couple à délivrer en sortie a atteint le couple prédéterminé,

le calcul du couple $\tau$ pour la première période selon une équation

$$\tau = \frac{t}{T1} \cdot \tau_f \cdot \alpha 1 + \left(1 - \frac{t}{T1}\right) \cdot \tau_s \cdot \beta 1$$

,

une valeur $\alpha 1$ indiquant un premier paramètre, une valeur $\beta 1$ indiquant un deuxième paramètre, une valeur t indiquant le temps écoulé dans le temps de commutation T1, une valeur $\tau_s$ indiquant un couple à un début du temps de commutation T1, et une valeur $\tau_f$ indiquant un couple à une fin du temps de commutation T1, la réinitialisation du premier paramètre $\alpha 1$ et du deuxième paramètre $\beta 1$, selon un taux de variation du couple dans la première période calculé par l'équation, et le calcul du couple $\tau$ pour la deuxième période selon l'équation en utilisant le premier paramètre $\alpha 1$ réinitialisé et le deuxième paramètre $\beta 1$ réinitialisé.

2. Dispositif de réduction de charge (21) selon la revendication 1, dans lequel le moyen de lissage de sortie de couple (2123) calcule le couple dans la première période obtenu par addition du couple d'appui $\tau_s$ et du couple de balancement $\tau_f$ selon le premier paramètre $\alpha 1$ et le deuxième paramètre $\beta 1$ pour le lissage et le rapport du temps écoulé t au temps de commutation T1, réinitialise le premier paramètre $\alpha 1$ et le deuxième paramètre $\beta 1$ utilisé pour le lissage dans la deuxième période selon le taux de variation du couple calculé dans la première période pour calculer le couple dans la deuxième période en utilisant le premier paramètre $\alpha 1$ réinitialisé et le deuxième paramètre $\beta 1$ réinitialisé.

3. Dispositif de réduction de charge (21) selon la revendication 1 ou la revendication 2, dans lequel le moyen d'estimation de couple (2121) calcule le couple d'appui $\tau_s$ et le couple de balancement $\tau_f$ de chaque articulation sur la base d'un modèle de commande dans lequel chaque articulation sur le plan sagittal de l'utilisateur est liée.

4. Dispositif de réduction de charge (21) selon l'une quelconque de la revendication 1 à la revendication 3, dans lequel le moyen de détermination d'appui/de balancement (2122) détermine si l'état de chaque jambe est à l'état d'appui ou à l'état de balancement sur la base de la valeur des charges exercées sur les plantes de pied des jambes.

5. Procédé de réduction de charge comprenant :

l'acquisition, à des intervalles prédéterminés, de charges exercées sur les plantes de pied des jambes d'un utilisateur et des angles au niveau de chacune des articulations des jambes de l'utilisateur ; le calcul de manière séquentielle d'un couple d'appui délivré en sortie pendant une période d'appui et d'un couple de balancement délivré en sortie pendant une période de balancement au moyen d'un mécanisme d'entraînement pour délivrer en sortie un couple afin de réduire une charge exercée sur l'utilisateur au niveau des articulations des jambes, sur la base de valeurs des charges exercées sur les plantes de pied des jambes et des angles au niveau de chacune des articulations des jambes qui sont acquises aux intervalles prédéterminés ; le fait de déterminer si chacune des jambes est dans un état d'appui ou un état de balancement ; et lors d'une commutation dans un état de chacune des jambes, le lissage d'une transition dans le couple délivré en sortie par le mécanisme d'entraînement dans un temps de commutation T1, conformément à un temps écoulé t sur la base du couple d'appui $\tau_s$ et du couple de balancement $\tau_f$ qui sont calculés séquentiellement, dans lequel le lissage comporte :

la fourniture du temps de commutation T1 pour commuter entre une période au cours de laquelle le mécanisme d'entraînement délivre en sortie le couple d'appui $\tau_s$ et une période au cours de laquelle le mécanisme d'entraînement délivre en sortie le couple de balancement $\tau_f$, le temps de commutation T1 comportant une première période de temps jusqu'à ce que le couple à délivrer en sortie atteigne un couple prédéterminé indiquant la moitié du couple d'appui $\tau_s$, et une deuxième période de temps après que le couple à délivrer en sortie a atteint le couple prédéterminé ; le calcul du couple $\tau$ pour la première période selon une équation

$$\tau = \frac{t}{T1} \cdot \tau_f \cdot \alpha 1 + \left(1 - \frac{t}{T1}\right) \cdot \tau_s \cdot \beta 1 \quad ,$$

une valeur $\alpha 1$ indiquant un premier paramètre; une valeur $\beta 1$ indiquant un deuxième paramètre, une valeur t indiquant le temps écoulé dans le temps de commutation T1, une valeur $\tau_s$ indiquant un couple à un début du temps de commutation T1, et une valeur $\tau_f$ indiquant un couple à une fin du temps de commutation T1 ;

la réinitialisation du premier paramètre $\alpha 1$ et du deuxième paramètre $\beta 1$, selon un taux de variation du couple dans la première période calculé par l'équation ; et le calcul du couple $\tau$ pour la deuxième période selon l'équation en utilisant le premier paramètre $\alpha 1$ réinitialisé et le deuxième paramètre $\beta 1$ réinitialisé.

**6.** Programme qui amène un ordinateur d'un dispositif de réduction de charge à exécuter des processus, les processus comprenant :

l'acquisition, à des intervalles prédéterminés, de charges exercées sur les plantes de pied des jambes d'un utilisateur et des angles au niveau de chacune des articulations des jambes de l'utilisateur ;
le calcul de manière séquentielle d'un couple d'appui délivré en sortie pendant une période d'appui et d'un couple de balancement délivré en sortie pendant une période de balancement au moyen d'un mécanisme d'entraînement pour délivrer en sortie un couple afin de réduire une charge exercée sur l'utilisateur au niveau des articulations des jambes, sur la base de valeurs des charges exercées sur les plantes de pied des jambes et des angles au niveau de chacune des articulations des jambes qui sont acquises aux intervalles prédéterminés ;
le fait de déterminer si chacune des jambes est dans un état d'appui ou un état de balancement ; et
lors d'une commutation dans un état de chacune des jambes, le lissage d'une transition dans le couple délivré en sortie par le mécanisme d'entraînement dans un temps de commutation T1, conformément à un temps écoulé t sur la base du couple d'appui $\tau_s$ et du couple de balancement $\tau_f$ qui sont calculés séquentiellement,
dans lequel le lissage comporte :

la fourniture du temps de commutation T1 pour commuter entre une période au cours de laquelle le mécanisme d'entraînement délivre en sortie le couple d'appui $\tau_s$ et une période au cours de laquelle le mécanisme d'entraînement délivre en sortie le couple de balancement $\tau_f$,
le temps de commutation T1 comportant une première période de temps jusqu'à ce que le couple à délivrer en sortie atteigne un couple prédéterminé indiquant la moitié du couple d'appui $\tau_s$, et une deuxième période de temps après que le couple à délivrer en sortie a atteint le couple prédéterminé ;
le calcul du couple $\tau$ pour la première période selon une équation

$$\tau = \frac{t}{T1} \cdot \tau_f \cdot \alpha 1 + \left(1 - \frac{t}{T1}\right) \cdot \tau_s \cdot \beta 1$$

une valeur $\alpha 1$ indiquant un premier paramètre; une valeur $\beta 1$ indiquant un deuxième paramètre, une valeur t indiquant le temps écoulé dans le temps de commutation T1, une valeur $\tau_s$ indiquant un couple à un début du temps de commutation T1, et une valeur $\tau_f$ indiquant un couple à une fin du temps de commutation T1 ;
la réinitialisation du premier paramètre $\alpha 1$ et du deuxième paramètre $\beta 1$, selon un taux de variation du couple dans la première période calculé par l'équation ; et
le calcul du couple $\tau$ pour la deuxième période selon l'équation en utilisant le premier paramètre $\alpha 1$ réinitialisé et le deuxième paramètre $\beta 1$ réinitialisé.

FIG. 1

FIG. 2

FIG. 3

21

POWER SUPPLY UNIT 214

INTEGRATED CONTROL UNIT 212

TORQUE ESTIMATION UNIT 2121

STANCE/SWING DETERMINATION UNIT 2122

TORQUE OUTPUT SMOOTHING 2123

INFORMATION ACQUISITION UNIT 211

ACTUATOR CONTROL UNIT 213

HIP JOINT SENSOR 23

KNEE JOINT SENSOR 24

ANKLE JOINT SENSOR 25

FOOT SOLE LOAD SENSOR 18

HIP ACTUATOR 13

KNEE ACTUATOR 14

ANKLE ACTUATOR 15

## FIG. 4

FIG. 5

UPWARD DIRECTION

FORWARD DIRECTION

$Fcos\theta_A\,cos\theta_K$

$Fcos\theta_A$

$\theta_K$

$\tau_3$

$Fcos\theta_A\,sin\theta_K$

$\tau_2$

$F$

$\theta_A$

$Fsin\theta_A$

$\tau_1$

## FIG. 6

**(A)**

| WALKING | BOTH LEGS STANCE | ONE LEG STANCE | BOTH LEGS STANCE | ONE LEG SWING |
|---|---|---|---|---|

| RUNNING | ONE LEG STANCE | BOTH LEGS SWING | ONE LEG SWING | BOTH LEGS SWING |
|---|---|---|---|---|

**(B)**

| STATE TRANSITION | | WHEN BOTH LEGS ARE STANCE | WHEN ONE LEG IS STANCE /ONE LEG IS SWING | WHEN BOTH LEGS ARE SWING |
|---|---|---|---|---|
| | | STANCE  STANCE | SWING  STANCE | SWING  SWING |
| SENSOR | HIP JOINT | JOINT ANGLE FROM ENCODER | | |
| | KNEE JOINT | | | |
| | ANKLE JOINT | | | |
| | FOOT SOLE LOAD | FOOT SOLE LOAD SENSOR GROUND CONTACT RECOGNITION FOR BOTH FEET | STANCE SIDE: FOOT SOLE LOAD SENSOR GROUND CONTACT RECOGNITION SWING SIDE: FOOT SOLE LOAD SENSOR GROUND NON-CONTACT RECOGNITION | FOOT SOLE LOAD SENSOR GROUND NON-CONTACT RECOGNITION FOR BOTH FEET |
| ACTUATOR | WAIST | DRIVEN WITH BOTH LEGS IN THE STANCE MODE | DRIVEN WITH BOTH LEGS IN THE SWING MODE | |
| | KNEE | | STANCE SIDE: DRIVEN IN STANCE MODE SWING SIDE: DRIVEN IN SWING MODE | DRIVEN WITH BOTH LEGS IN THE SWING MODE |
| | ANKLE | DRIVEN WITH BOTH LEGS IN THE STANCE MODE | STANCE SIDE: DRIVEN IN STANCE MODE SWING SIDE: DRIVEN IN SWING MODE | SWING SIDE: DRIVEN IN SWING MODE |

FIG. 7

FIG. 8

FIG. 9

```
                    ┌──────────────┐
                    │    START     │
                    └──────┬───────┘
                           │
        ┌──────────────────▼──────────────────┐
    ┌──▶│   ACQUIRE JOINT ANGLE INFORMATION   │──── S101
    │   └──────────────────┬──────────────────┘
    │   ┌──────────────────▼──────────────────┐
    │   │  ACQUIRE FOOT SOLE LOAD INFORMATION │──── S102
    │   └──────────────────┬──────────────────┘
    │   ┌──────────────────▼──────────────────┐
    │   │           ESTIMATE TORQUE           │──── S103
    │   └──────────────────┬──────────────────┘
    │   ┌──────────────────▼──────────────────┐
    │   │           STANCE/SWING              │──── S104
    │   │           DETERMINATION             │
    │   └──────────────────┬──────────────────┘
    │   ┌──────────────────▼──────────────────┐
    │   │       TORQUE OUTPUT SMOOTHING       │──── S105
    │   └──────────────────┬──────────────────┘
    │   ┌──────────────────▼──────────────────┐
    │   │      OUTPUT TORQUE FROM ACTUATORS   │──── S106
    │   └──────────────────┬──────────────────┘
    └───────────────────────┘
```

FIG. 10

```
┌─────────────────────────────────────────┐
│                                      ┌─ 21
│  ┌───────────────────────────────┐   │
│  │     TORQUE ESTIMATION UNIT    │── 2121
│  └───────────────────────────────┘   │
│  ┌───────────────────────────────┐   │
│  │        STANCE/SWING           │── 2122
│  │     DETERMINATION UNIT        │   │
│  └───────────────────────────────┘   │
│  ┌───────────────────────────────┐   │
│  │       TORQUE OUTPUT           │── 2123
│  │      SMOOTHING UNIT           │   │
│  └───────────────────────────────┘   │
│                                       │
└─────────────────────────────────────────┘
```

**EP 3 885 081 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015144787 A
- JP 2017099798 A
- JP 2013138848 A

- WO 2010011848 A1
- KR 101317354 B
- JP 2018219220 A